# EUROPEAN PATENT APPLICATION

(11) **EP 3 139 191 A1**
(43) Date of publication of application: **08.03.2017**
(21) Application number: 16187173.6
(22) Date of filing: 05.09.2016
(51) Int. Cl.: G01R 33/12, A61B 5/05, G01N 24/08, G01R 33/36

(54) **APPARATUS AND METHOD FOR DETECTING NONLINEAR MAGNETIC PARTICLE BASED ON SINGLE EXCITATION COIL**

(30) Priority: 07.09.2015 KR 20150126516; 11.04.2016 KR 20160044172
(71) Applicant: Electronics and Telecommunications Research Institute, Daejeon 34129 (KR)
(72) Inventor: CHOI, Seung-Min, 34071 Daejeon (KR); HONG, Hyo-Bong, 34140 Daejeon (KR)
(74) Representative: Betten & Resch

(57) **Abstract**

Disclosed are apparatus and method for detecting a nonlinear magnetic particle based on a single excitation coil. The apparatus includes a signal generation unit for generating an input composite signal by combining a high-frequency sine wave signal and a low-frequency sine wave signal, generated based on a fundamental frequency, with each other, a signal application unit for applying the input composite signal to a single excitation solenoid coil included in a measurement head, through which a sample passes, to generate a magnetic field in the measurement head so as to detect a nonlinear magnetic particle, and a detection unit for detecting an output signal emitted from the sample depending on the magnetic field by using at least one detection solenoid coil included in the measurement head, analyzing a frequency domain of the output signal, and detecting whether the nonlinear magnetic particle is present in the sample.

## Description

### BACKGROUND OF THE INVENTION

### 1. Technical Field

The present invention generally relates to Frequency Mixing Magnetic Detection (FMMD) technology and, more particularly, to an apparatus and method for detecting a nonlinear magnetic particle based on a single excitation coil, which can improve the performance of detection of a nonlinear magnetic particle and can reduce production costs when FMMD is implemented.

### 2. Description of the Related Art

The field technically closest to post-Magnetic Resonance Imaging (MRI) scanners, among pieces of medical imaging equipment based on magnetic properties, is a Magnetic Particle Imaging (MPI) system. MPI technologies developed to date differ greatly from the physical principle of existing MRI scanners. Actual MPI itself has the meaning of a magnetic particle analysis imaging system, but this technology is not limited to particles, and may be applied to a great variety of fields.

The first theory used in MPI technology is based on a Langevin equation proposed by the French scientist Paul Langevin. Actually, the Langevin equation theory was proposed to describe Brownian motion, and Paul Langevin could not have imagined that the Langevin equation theory would be used to acquire medical images.

The basic concept of MPI is based on two physical properties: the first property is that a specific magnetic (super-paramagnetic or paramagnetic in a specific situation) material has a nonlinear magnetic property. Carbon-based compounds, which are materials constituting most of the human body, are diamagnetic materials, which are slightly repelled by a magnetic field rather than being magnetized. Further, a magnetic material such as iron tends to exhibit magnetic force for a predetermined period of time when it is magnetized. However, when a typical magnetic material is reduced to a predetermined size of 50 nm to 100 nm or less, it has magnetism only when a magnet is positioned beside it. In this case, a super-paramagnetic material has a nonlinear magnetic property.

The second property is that a Field Free Point (FFP) or a Field Free Line (FFL) occurs in a Maxwell magnet. However, it is anticipated that it will take a lot of time to actually apply this concept to medical imaging equipment. Basic physical concepts appeared in the early 20th century, but the actual application thereof to medical imaging was initially attempted by scientists in the Royal Philips Research Lab located in Hamburg, Germany, in 2001, and the results of research conducted up to that time were gathered and finally published in Nature in 2005, and thus FFP and FFL have attracted attention all over the world.

Depending on the results of research conducted to date, there are advantages in that, when MPI is used, it is possible to acquire images having a high resolution of 0.4 cm, which cannot be achieved using only existing MRI, and in that, when FFP and FFL are electromagnetically moved, images may be acquired within a time of tens of ms. Further, there is an advantage in that, when various types of antigens-antibodies are coupled to magnetic particles used as a contrast medium, results which are non-radiological and are similar to those acquired from Positron Emission Tomography (PET) may be obtained.

Thereafter, as various advantages of MPI have become widely known, several research groups have started to introduce pieces of equipment which are slightly modified and to publish experimental results thereof.

As European research laboratories in which innovative research in related fields is conducted, the Royal Philips Research Laboratory and the University of Leubeck, which may be regarded as the originators, have yielded the greatest number of research results in the field of imaging based on FFL and FFP. Also, the German Helmholtz research center has developed next-generation medical imaging equipment based on various physical theories. For commercialization of this imaging equipment, EU companies may be considered to be the most technologically advanced in this field. At the present time, the size of equipment is implemented to such an extent that only small experimental animals can be measured, but the German universal research equipment production company released prototype MPI equipment for the first time in the world in collaboration with Philips in 2013.

In U.S., the Berkeley Imaging System Laboratory has most actively conducted research activities, and currently proceeds with a project named "X-space". Currently, this group has developed technology capable of acquiring images in real time from even microvessels, from which it is very difficult to capture images using only existing MRI scanners, and a new concept of 3D image acquisition algorithms.

In Korea, Electronics and Telecommunications Research Institute (ETRI) has most actively conducted related research. Technology currently developed in Korea is similar to the above research groups with regard to the physical principle itself, but is greatly different from the research groups as to the fields in which signals are actually acquired. The above research groups must inevitably depend on a high-magnetic field having considerable power so as to generate and acquire signals. Compared to MRI scanners, a very small magnetic field generation device, for example, one having a size corresponding to several tenths or hundredths of the size of the existing magnetic field generation devices, is used, but a disadvantage arises in that the size of the equipment itself must be increased to the same size as MRI scanners in order to measure images of a human being.

In order to overcome this disadvantage, an ETRI research team uses Frequency Mixing Magnetic Detection (FMMD) technology. The ETRI research team uses, as the physical principle itself, a principle that was proposed by Paoli in the 1970s which is considered to be relatively recently proposed compared to other medical imaging equipment. Experimental results indicating that signals based on this technology can be used to analyze a super-paramagnetic material at a specific location were published by researchers from the German Helmholtz research center in 2006, and have been used for research into a super-paramagnetic property. By using this physical law, ETRI research teams have proven that this physical law and principle can be applied not only to super-paramagnetic materials, but also to paramagnetic materials, which are spatially dense.

In connection with this, Korean Patent Application Publication No. 10-2009-0060143 discloses a technology related to "Quantitative Detection Method of Biomolecules Using Magnetic Nano Particle and Frequency Mixing Magnetic Reader."

### SUMMARY OF THE INVENTION

Accordingly, the present invention has been made keeping in mind the above problems occurring in the prior art, and an object of the present invention is to reduce expenses required for the production of products by omitting an operation of delicately calibrating the ratio, geometric positions, etc. of two excitation solenoid coils when FMMD is implemented.

Another object of the present invention is to improve the accuracy of detection when FMMD for measuring the nonlinearity of a nonlinear material located in a magnetic field applied to a single excitation solenoid coil is implemented.

In accordance with an aspect of the present invention to accomplish the above objects, there is provided an apparatus for detecting a nonlinear magnetic particle based on a single excitation coil, including a signal generation unit for generating an input composite signal by combining a high-frequency sine wave signal and a low-frequency sine wave signal, generated based on a fundamental frequency, with each other; a signal application unit for applying the input composite signal to a single excitation solenoid coil included in a measurement head, through which a sample passes, to generate a magnetic field in the measurement head so as to detect a nonlinear magnetic particle; and a detection unit for detecting an output signal emitted from the sample depending on the magnetic field by using at least one detection solenoid coil included in the measurement head, for analyzing a frequency domain of the output signal, and for detecting whether the nonlinear magnetic particle is present in the sample.

The magnetic field may be a sum of a first magnetic field, generated in response to the high-frequency sine wave signal, and a second magnetic field, generated in response to the low-frequency sine wave signal.

The detection unit may check whether a harmonic peak is detected in the frequency domain, and determine that the nonlinear magnetic particle is present in the sample if the harmonic peak is detected.

The detection unit may be configured to, when a signal in which two frequencies are combined with each other is detected in a deformed shape in the frequency domain, check whether the harmonic peak is detected.

The signal generation unit may generate the input composite signal by combining the high-frequency sine wave signal with the low-frequency sine wave signal using a combiner that adds and combines two signals.

The signal generation unit may generate the input composite signal in such a way as to amplify a strength of the high-frequency sine wave signal and a strength of the low-frequency sine wave signal using a first amplifier and a second amplifier, respectively, to input the amplified sine wave signals to the combiner, and to amplify a strength of a combined signal output from the combiner using a third amplifier.

The combiner may correspond to any one of a Radio Frequency (RF) combiner, a summing amplifier, and electronic equipment, which correspond to characteristics of the input composite signal, wherein the electronic equipment enables an add operation on analog signals.

The at least one detection solenoid coil may be located in the measurement head at a position closer to a center of the measurement head than the single excitation solenoid coil.

In accordance with another aspect of the present invention to accomplish the above objects, there is provided a method for detecting a nonlinear magnetic particle based on a single excitation coil, including generating an input composite signal by combining a high-frequency sine wave signal and a low-frequency sine wave signal, generated based on a fundamental frequency, with each other; applying the input composite signal to a single excitation solenoid coil included in a measurement head, through which a sample required to detect a nonlinear magnetic particle passes, so as to generate a magnetic field in the measurement head; and detecting an output signal emitted from the sample depending on the magnetic field by using at least one detection solenoid coil included in the measurement head, analyzing a frequency domain of the output signal, and detecting whether the nonlinear magnetic particle is present in the sample.

The magnetic field may be a sum of a first magnetic field, generated in response to the high-frequency sine wave signal, and a second magnetic field, generated in response to the low-frequency sine wave signal.

Detecting the output signal may include checking whether a harmonic peak is detected in the frequency domain; and determining that the nonlinear magnetic particle is present in the sample if the harmonic peak is detected.

Checking whether a harmonic peak is detected may be configured to, when a signal in which two frequencies are combined with each other is detected in a deformed shape in the frequency domain, check whether the harmonic peak is detected.

Generating the input composite signal may be configured to generate the input composite signal by combining the high-frequency sine wave signal with the low-frequency sine wave signal using a combiner that adds and combines two signals.

Generating the input composite signal may include amplifying a strength of the high-frequency sine wave signal and a strength of the low-frequency sine wave signal using a first amplifier and a second amplifier, respectively, and inputting the amplified sine wave signals to the combiner; and amplifying a strength of a combined signal output from the combiner using a third amplifier, thus generating the input composite signal.

The combiner may correspond to any one of an RF combiner, a summing amplifier, and electronic equipment, which correspond to characteristics of the input composite signal, wherein the electronic equipment enables an add operation on analog signals.

The at least one detection solenoid coil may be located in the measurement head at a position closer to a center of the measurement head than the single excitation solenoid coil.

### BRIEF DESCRIPTION OF THE DRAWINGS

The above and other objects, features and advantages of the present invention will be more clearly understood from the following detailed description taken in conjunction with the accompanying drawings, in which:
FIG. 1 is a diagram showing a structure for measuring a nonlinear magnetic particle based on a Frequency Mixing Magnetic Detection (FMMD) technique;
FIG. 2 is a block diagram showing an apparatus for detecting a nonlinear magnetic particle based on a single excitation coil according to an embodiment of the present invention;
FIG. 3 is a diagram, showing a structure for measuring a nonlinear magnetic particle to which the nonlinear magnetic particle detection apparatus shown in FIG. 2 is applied;
FIG. 4 is a block diagram showing in detail the signal generation unit shown in FIG. 2, based on the structure of FIG. 3 ;
FIG. 5 is a diagram showing a measurement head shown in FIG. 1 and a measurement head according to an embodiment of the present invention;
FIGS. 6 to 10 are diagrams showing examples of the results of simulation of the nonlinear magnetic particle detection apparatus according to the present invention;
FIG. 11 is an operation flowchart showing a method for detecting a nonlinear magnetic particle based on a single excitation coil according to an embodiment of the present invention;
FIG. 12 is an operation flowchart showing in detail a method for detecting a nonlinear magnetic particle based on a single excitation coil according to an embodiment of the present invention; and
FIG. 13 is a circuit diagram showing an example of a summing amplifier according to an embodiment of the present invention.

### DESCRIPTION OF THE PREFERRED EMBODIMENTS

The present invention will be described in detail below with reference to the accompanying drawings. Repeated descriptions and descriptions of known functions and configurations which have been deemed to make the gist of the present invention unnecessarily obscure will be omitted below. The embodiments of the present invention are intended to fully describe the present invention to a person having ordinary knowledge in the art to which the present invention pertains. Accordingly, the shapes, sizes, etc. of components in the drawings may be exaggerated to make the description clearer.

Hereinafter, preferred embodiments of the present invention will be described in detail with reference to the attached drawings.

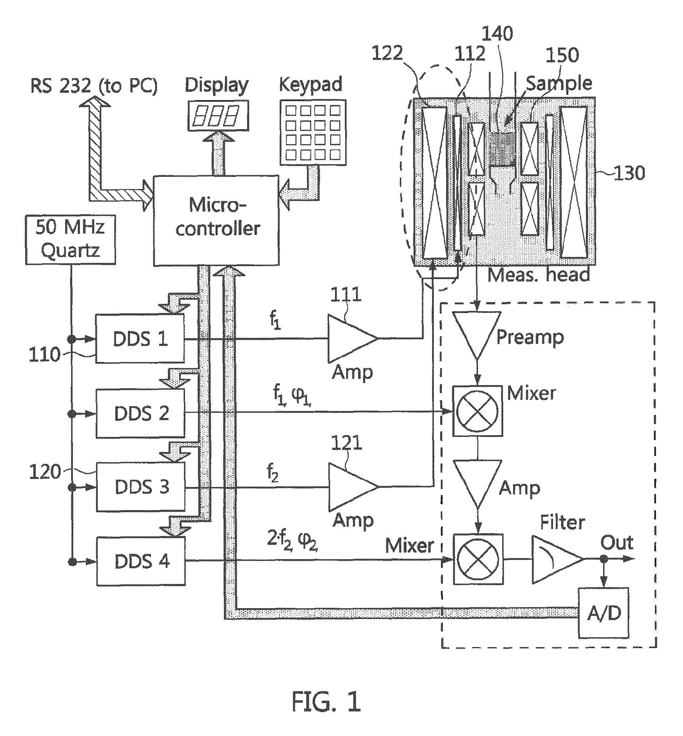

FIG. 1 is a diagram showing a structure for measuring a nonlinear magnetic particle based on an FMMD technique.

Referring to FIG. 1, in the structure for measuring a nonlinear magnetic particle based on an FMMD technique, "meas, head" may mean a measurement head, that is, a measurement head 130.

Here, it can be seen that the inner part of the measurement head 130 is composed of solenoid coils stacked as multiple layers, as shown in FIG. 1.

A low-frequency excitation solenoid coil 122 for performing excitation at a second frequency, that is, a low frequency, which is generated by a low-frequency generation module 120 and is amplified by an amplifier 121, in a sample 140, may be located in the outermost layer of the measurement head 130.

Further, a high-frequency excitation solenoid coil 112 for performing excitation at a first frequency, that is, a high frequency, which is generated by a high-frequency generation module 110 and is amplified by an amplifier 111, in the sample 140, may be located in the next-to-outermost layer (i.e. further inside than the low-frequency excitation solenoid coil 122).

Furthermore, a detection solenoid coil 150 for detecting an output signal emitted from the sample 140 may be located in the third-to-outermost layer (i.e. further inside than the high-frequency excitation solenoid coil 112), and the sample 140 for which the presence or absence of a nonlinear magnetic particle is to be determined may be located at the center of the measurement head.

Here, referring to the dotted ellipse shown in FIG. 1, two excitation solenoid coils are used to perform excitation and produce signals, that is, frequencies. When this type of product is produced, an operation of delicately calibrating the ratio, the geometric positions, or the like of two excitation solenoid coils is essentially required, which may be a cause of increasing expenses.

Therefore, to solve this problem, the present invention intends to propose a structure for removing one excitation solenoid coil and for allowing an electronic device, such as a combiner, to combine signals with each other in advance and to provide a composite signal in order to generate the total sum of magnetic fields generated by two excitation solenoid coils instead of the removed coil.

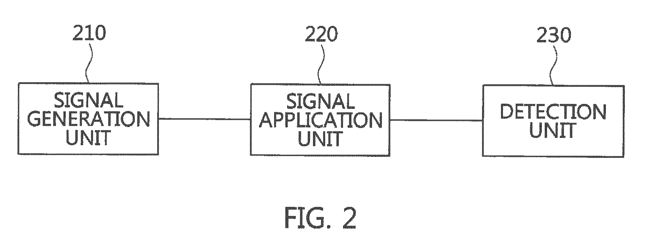

FIG. 2 is a block diagram showing an apparatus for detecting a nonlinear magnetic particle based on a single excitation coil according to an embodiment of the present invention.

Referring to FIG. 2, the apparatus for detecting a nonlinear magnetic particle based on a single excitation coil according to the embodiment of the present invention may include a signal generation unit 210, a signal application unit 220, and a detection unit 230.

The signal generation unit 210 may generate an input composite signal by combining a high-frequency sine wave signal and a low-frequency sine wave signal, which are generated based on a fundamental frequency, with each other.

Here, the fundamental frequency may correspond to a fundamental waveform that is indicated by a sine function and is used to indicate a spatial frequency or sound.

Here, before the input signal is applied to the excitation solenoid coil, the input composite signal is generated by combining two signals, and thus the procedure for performing calibration using two excitation solenoid coils in the structure of FIG. 1 may be omitted. That is, there is an advantage in that the expense incurred for one excitation solenoid coil and the expense incurred for calibration may be reduced.

In this case, by using a combiner for adding and combining two signals, a high-frequency sine wave signal and a low-frequency sine wave signal may be combined with each other, and then an input composite signal may be generated.

Here, the combiner may be a passive electronic element for adding and combining two signals. That is, by adding and combining two signals, they may be combined with each other without mutually influencing each other.

The combiner may correspond to any one of a Radio Frequency (RF) combiner, corresponding to the characteristics of the input composite signal, a summing amplifier, and electronic equipment enabling an add operation on analog signals.

Here, the RF combiner, which is a kind of passive circuit, may mean a circuit for equally or differentially distributing the power of a specific signal or for combining the power. Here, the RF combiner may add and combine two signals, unlike a mixer for mixing two frequencies with each other and detecting only a frequency signal corresponding to the difference between the two frequencies.

The summing amplifier may be a circuit for performing only an add function, among the functions of an existing operational amplifier for adding or subtracting multiple signals, or differentiating or integrating the signals.

Here, as the combiner of the present invention, which one of the RF combiner and the summing amplifier is to be used may be set by a user or a manager who implements FMMD.

Further, the combiner may be a concept including not only electronic equipment that is conventionally used, such as an RF combiner or a summing amplifier, but also all electronic equipment that may be developed in the future and enables an add operation on analog signals.

Here, the strength of the high-frequency sine wave signal and the strength of the low-frequency sine wave signal are respectively amplified using a first amplifier and a second amplifier, and the amplified sine wave signals are input to the combiner. The strength of the combined signal, output from the combiner, is amplified using a third amplifier, and thus the input composite signal may be generated.

Here, the first amplifier and the second amplifier may adjust and amplify the strengths of the high-frequency sine wave signal and the low-frequency sine wave signal, respectively, depending on the characteristics of the input composite signal. By differently amplifying the strengths of respective signals, the effect of controlling the characteristics of an input signal may be implemented by calibrating the ratio and the geometric positions of two excitation solenoid coils in the conventional technology.

Further, the third amplifier may adjust the strength of the signal in response to loss that occurs in the procedure for combining two signals through the combiner, which is a passive electronic element. That is, the strength of the combined signal output from the combiner may be amplified in response to the loss of strength occurring in the combiner.

The signal application unit 220 may apply the input composite signal to a single excitation solenoid coil included in the measurement head through which a sample passes, to generate a magnetic field in the measurement head in order to detect a nonlinear magnetic particle.

Here, "nonlinear magnetic particle" may literally mean a material or a particle having a nonlinear magnetic property. That is, the nonlinear magnetic particle may be a material for making a response that is not proportional to the strength or intensity of the magnetic field generated in the single excitation solenoid coil.

Here, the measurement head may be composed of several layers, wherein the single excitation solenoid coil may be located in the outermost layer of the measurement head. Further, the sample may be influenced by the magnetic field generated in the single excitation solenoid coil while passing through the center portion of the measurement head.

The detection unit 230 may detect an output signal emitted from the sample depending on the magnetic field using at least one detection solenoid coil included in the measurement head, and may detect whether a nonlinear magnetic particle is present in the sample by analyzing the frequency domain of the output signal.

Here, the magnetic field may be the sum of a first magnetic field, generated in response to the high-frequency sine wave signal, and a second magnetic field, generated in response to the low-frequency sine wave signal. That is, in the structure shown in FIG. 1, when two excitation solenoid coils respectively generate magnetic fields and the magnetic fields are added with each other by calibrating the geometric positions of the two coils, the present invention may obtain the same effect that is obtained by applying a signal, in which the two signals are combined with each other, to a single excitation solenoid coil.

Whether a harmonic peak is detected in the frequency domain is checked. When a harmonic peak is detected, it may be determined that a nonlinear magnetic particle is present in the sample.

Such a harmonic peak, which is a frequency peak corresponding to a specific frequency, may be detected when a nonlinear magnetic particle is present in the sample. Here, the characteristics of the corresponding particle may also be grasped based on the harmonic peak detected in the frequency domain.

When a signal in which two frequencies are combined with each other is detected in a deformed shape in the frequency domain, it may be checked whether a harmonic peak is detected. That is, when a nonlinear magnetic particle is present in the sample, the output signal emitted from the sample may be detected in a form that is not proportional to the combined signal of two frequencies.

Therefore, when a deformed signal that is not proportional to the combined signal of two frequencies is detected in this way, it may be inferred that a nonlinear magnetic particle is present in the sample, and the detection of the harmonic peak may be performed.

Here, at least one detection solenoid coil may be located closer to the center of the measurement head than the single excitation solenoid coil in the measurement head. Therefore, the single excitation solenoid coil may be located in the outermost layer of the measurement head, at least one detection solenoid coil may be located further inside than the single excitation solenoid coil, and a space through which the sample passes may be present at the center of the measurement head, which corresponds to an area further inside than the detection solenoid coil.

The above-described nonlinear magnetic particle detection apparatus is used in this way, so that, when FMMD is implemented, an operation of delicately calibrating the ratio of two excitation solenoid coils, the geometric positions of the coils, etc. may be omitted, thus reducing expenses when products are produced.

Further, when the FMMD for measuring the nonlinearity of a nonlinear material located in the magnetic field applied to the single excitation solenoid coil is implemented, the accuracy of detection may be improved.

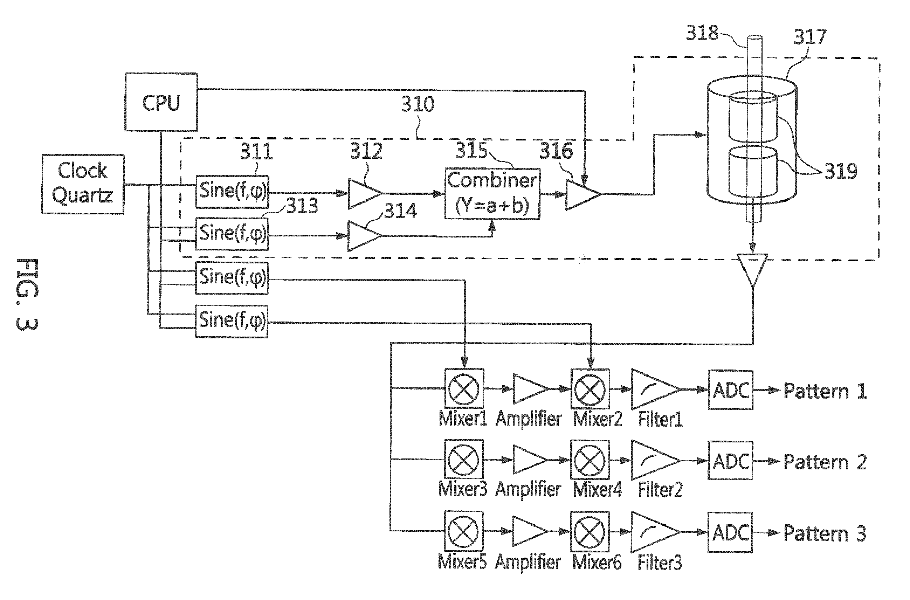

FIG. 3 is a diagram showing a structure for measuring a nonlinear magnetic particle, to which the nonlinear magnetic particle detection apparatus shown in FIG. 2 is applied.

Referring to FIG. 3, it can be seen that, in the nonlinear magnetic particle measurement structure to which the nonlinear magnetic particle detection apparatus shown in FIG. 2 is applied, one of two excitation solenoid coils used for excitation of a frequency in a sample in FIG. 1 is removed. Further, instead of the removed excitation solenoid coil, a combiner is included so as to combine an input signal and provide a combined signal, and one additional amplifier for adjusting the strength of the combined signal, output from the combiner, is added, in contrast with the scheme shown in FIG. 1.

Consequently, although one excitation solenoid coil is omitted, and one combiner and one amplifier are added, the expense saved when one excitation solenoid coil is omitted is greater than the expense incurred due to the addition of the combiner and the amplifier, and thus this structure may be regarded as a more efficient structure than that of FIG. 1.

In the measurement structure shown in FIG. 3, the nonlinear magnetic particle detection apparatus according to the present invention may be operated to correspond to a detection apparatus area 310. Therefore, for help in understanding of the present invention, the procedure for detecting a nonlinear magnetic particle will be described based on the detection apparatus area 310 using the structure of FIG. 3 as an embodiment.

First, in order to detect whether a nonlinear magnetic particle is present in a specific sample, there is a need to generate an input signal to be applied to a measurement head. Here, in the nonlinear magnetic particle detection apparatus according to the present invention, the input signal may be an input composite signal. For this, a fundamental frequency, generated by a clock quartz oscillator included in the measurement structure of FIG. 3, may be individually provided to a high-frequency generation module 311 and to a low-frequency generation module 313, thus enabling a high-frequency sine wave signal and a low-frequency sine wave signal to be generated.

Here, the low-frequency generation module 313 may receive a separate set value based on a Central Processing Unit (CPU) included in the measurement structure, and may generate a low frequency by modulating the fundamental frequency using the set value.

Thereafter, the high-frequency sine wave signal and the low-frequency sine wave signal may be input to a combiner 315 through respective amplifiers 312 and 314.

Here, the two amplifiers 312 and 314 may amplify the high-frequency sine wave signal and the low-frequency sine wave signal by adjusting the strengths thereof depending on the characteristics of the input composite signal to be input to a single excitation solenoid coil 317.

That is, in the technology shown in FIG. 1, if the frequency for excitation in the sample is controlled via the calibration of the ratio and geometric positions of two excitation solenoid coils. However, the present invention must control the frequency in advance during the procedure for combining two signals with each other, and thus the strengths of signals input to the combiner 315 through the amplifiers 312 and 314 may be respectively adjusted.

Thereafter, the combiner 315 may function to add and combine two signals input from the amplifiers 312 and 314.

Here, the term "adding and combining" may mean that two signals are combined with each other without being changed and influencing each other, rather than interacting with each other and generating a modulated signal. That is, "adding and combining" may be different from typical "mixing", in which a frequency component corresponding to the sum or difference between two signals is generated using multiplication.

Here, the combiner 315 may be a passive electronic element, and thus loss may occur in the procedure for combining two signals through the combiner 315.

Therefore, after two signals have been combined with each other through the combiner 315, the strength of a combined signal output from the combiner 315 is adjusted via an amplifier 316, and then an input composite signal may be generated.

Thereafter, the input composite signal may be applied to the single excitation solenoid coil 317 located in the outermost layer of the measurement head.

Here, the single excitation solenoid coil 317 may generate a type of magnetic field in which two frequencies are combined with each other in response to the input composite signal.

The excitation solenoid coil may correspond to an analog part of a measuring sensor based on the FMMD technique, and may be an important factor in determining a Signal-to-Noise Ratio (SNR). Here, the excitation solenoid coil may greatly influence the sensitivity of detection depending on the thickness of a conducting wire, the number of windings of the coil, or the like. Moreover, in the scheme of FIG. 1, there is essentially required a calibration procedure for calibrating the geometric positions of two excitation solenoid coils.

However, in the present invention, a calibration operation which is performed in the scheme of FIG. 1 is processed using the combiner 315, which is a passive electronic element, and thus coil production and calibration expenses may be reduced. Further, when the scheme of the present invention is used, the development of FMMD is expected to be more easily performed in the future.

Here, the sample may be influenced by the magnetic field of the single excitation solenoid coil 317 while passing through a sample transmission path 318 located at the center of the measurement head. In this regard, when a nonlinear magnetic particle is present in the sample, a signal in which a magnetic field is deformed by the nonlinear magnetic particle may be detected from the output signal emitted from the sample. Further, when the frequency domain of the signal deformed by the nonlinear magnetic particle is checked, a harmonic peak may be detected.

Therefore, when a harmonic peak is detected from the output signal emitted from the sample, it may be determined that a nonlinear magnetic particle is present in the sample.

Here, the remaining modules of the measurement structure shown in FIG. 3, which are not included in the detection apparatus area 310, may not fall within the scope of the present invention, but they are depicted as being included in FIG. 3 for the convenience of description.

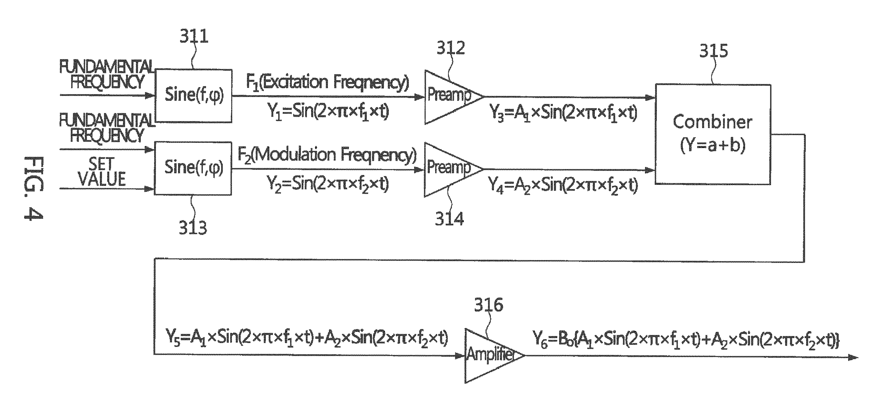

FIG. 4 is a block diagram showing in detail the signal generation unit shown in FIG. 2, based on the structure of FIG. 3.

Referring to FIG. 4, the signal generation unit may include a high-frequency generation module 311 for generating a high-frequency sine wave signal corresponding to Y₁ = sin(2*π*f₁*t) based on a fundamental frequency.

Here, a sine wave is indicated by a sine function and may correspond to a fundamental waveform used to indicate a spatial frequency or sound. Further, 2*π*f₁ may mean an angular frequency ω of frequency f₁, and t may mean a period.

Thereafter, an amplifier (preamplifier) 312 may amplify the strength of the signal Y₁ to a level corresponding to A₁.

Here, the signal obtained by amplifying the strength of the signal Y₁ may be Y₃ = A₁ *sin(2*π*f₁*t).

Further, the signal generation unit shown in FIG. 2 may include a low-frequency generation module 313 for generating a low-frequency sine wave signal corresponding to Y₂ = sin(2*π*f₂*t) based on the fundamental frequency.

Thereafter, an amplifier (preamplifier) 314 may amplify the strength of the signal Y₂ to a level corresponding to A₂.

Here, the signal obtaining by amplifying the strength of the signal Y₂ may be Y₄ = A₂*sin(2*π*f₂*t).

Thereafter, a combiner 315 may add and combine the signals Y₃ and Y₄, generated through the amplifiers 312 and 314. That is, a signal Y₅, generated by combining the two signals with each other, may be Y₅ = A₁*sin(2*π*f₁*t)+ A₂*sin(2*π*f₂*t).

Here, since the combiner 315 may be a passive electronic element, loss may occur in the procedure for combining signals.

Therefore, the combined signal Y₅, generated through the combiner 315, may be additionally amplified to a strength corresponding to Bo through an amplifier 316, and then an input composite signal may be generated.

The input composite signal, which has been amplified through the amplifier 316, may be Y₆ = B₀{A₁*sin(2*π*f₁*t) + A₂*sin(2*π*f₂*t)}.

The input composite signal Y₆ generated in this way may be applied to a single excitation solenoid coil included in the measurement head through the signal application unit shown in FIG. 2.

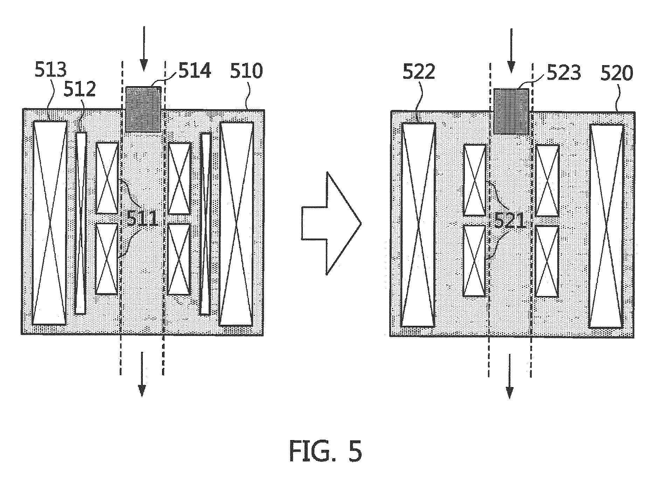

FIG. 5 is a diagram showing the measurement head shown in FIG. 1 and the measurement head according to an embodiment of the present invention.

Referring to FIG. 5, the section of a measurement head 510 shown in FIG. 1 and the section of a measurement head 520 that is modified according to an embodiment of the present invention may be shown.

In this case, it can be seen that two excitation solenoid coils 512 and 513 for performing excitation to produce signals in a sample are present in the measurement head 510 shown in FIG. 1, whereas only a single excitation solenoid coil 522 is present in the modified measurement head 520.

Here, the measurement head 510 shown in FIG. 1 must require an operation of calibrating the geometric positions of the two excitation solenoid coils 512 and 513 so as to produce signals in a sample 514 using the excitation solenoid coils 512 and 513. However, since the position calibration operation requires very delicate calibration, this may be the cause of increasing production costs and production time when products are produced. Further, there may occur differences between detection results for respective products depending on whether position calibration is precise.

Therefore, the present invention intends to present technology capable of performing excitation to produce a signal in a sample 523 using only a single excitation solenoid coil 522, as in the case of the modified measurement head 520 shown in FIG. 5, and detecting whether a nonlinear magnetic particle is present in the sample 523.

That is, an operation of applying signals to the sample 514 using the two excitation solenoid coils 512 and 513 in the measurement head 510 shown in FIG. 1 may be processed using only the single excitation solenoid coil 522 in the modified measurement head 520.

For this, an input composite signal, in which two signals are combined with each other through the combiner, may be applied to the single excitation solenoid coil 522 included in the modified measurement head 520. Here, the two signals may be combined with each other in a manner corresponding to an add operation. Even after the two signals have been combined with each other, they may not change, and thus may not influence each other.

Therefore, the single excitation solenoid coil 522 included in the modified measurement head 520 may generate a magnetic field in which magnetic fields corresponding to two signals included in an input composite signal are combined with each other when the input composite signal is applied.

The present invention detects whether a nonlinear magnetic particle is present in the sample 523 using the modified measurement head 520 in this way, thus resulting in the advantage of reducing production costs for excitation solenoid coils and the expense incurred for the position calibration operation.

Here, the two excitation solenoid coils 512 and 513, included in the measurement head 510 shown in FIG. 1, and the single excitation solenoid coil 522, included in the modified measurement head 520, may be produced such that conducting wires thereof have different thicknesses and such that corresponding coils have different numbers of windings.

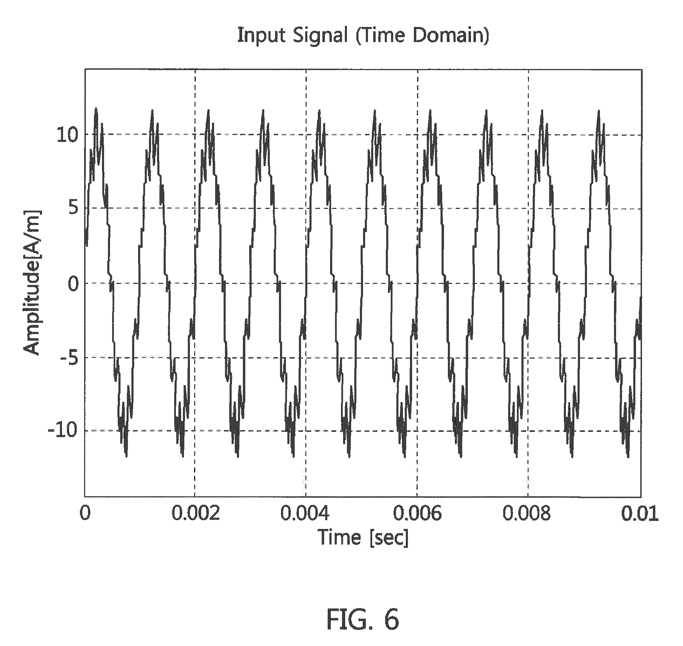

FIGS. 6 to 10 are diagrams showing examples of the results of simulation using the nonlinear magnetic particle detection apparatus according to the present invention.

Referring to FIGS. 6 to 10, FIG. 6 may show that an input composite signal, generated to detect a nonlinear magnetic particle, is detected according to the present invention.

Here, since the input composite signal is obtained by adding and combining a high-frequency sine wave signal and a low-frequency sine wave signal, the input composite signal may be detected in a form in which a frequency corresponding to the high-frequency sine wave signal and a frequency corresponding to the low-frequency sine wave signal are added.

Thereafter, to detect a nonlinear magnetic particle, the input composite signal may be applied to a single excitation solenoid coil included in the measurement head, and thus a magnetic field may be generated.

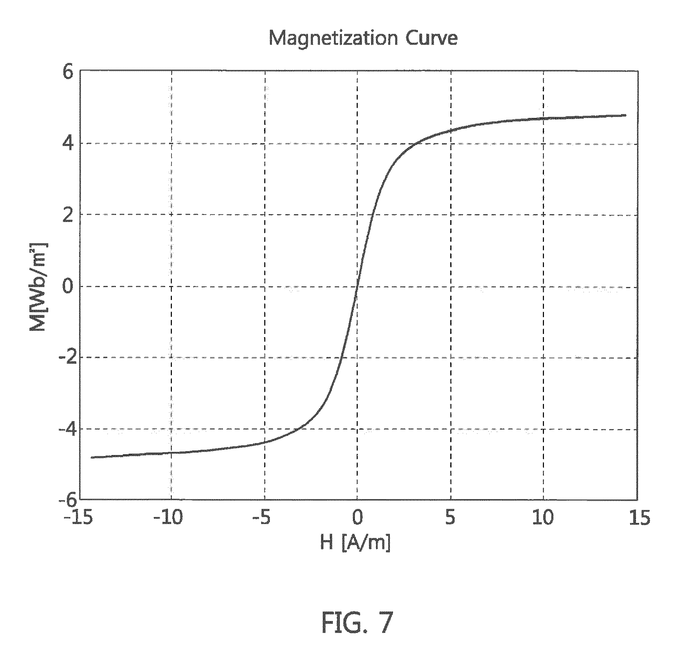

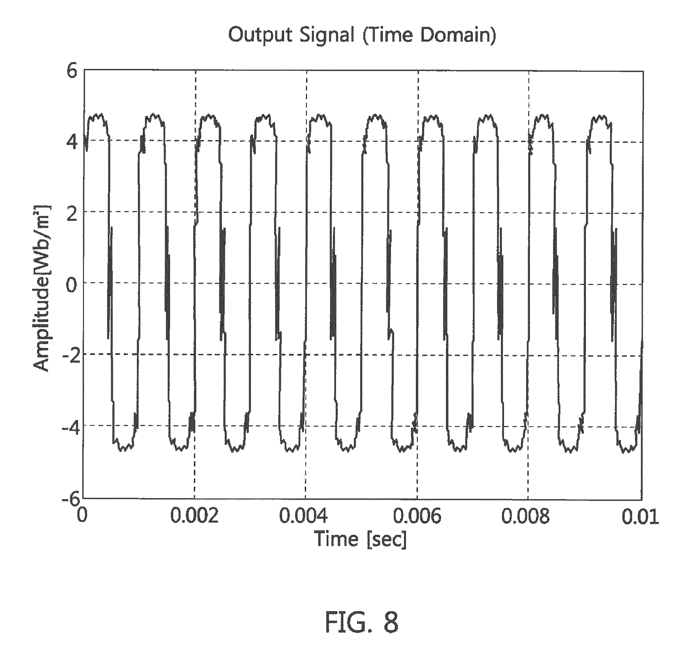

Here, assuming that a nonlinear magnetic particle causing the signal, such as that shown in FIG. 7, is present in the sample, an output signal having the shape, such as that shown in FIG. 8, may be detected via a detection solenoid coil included in the measurement head. That is, the input composite signal having the shape, such as that shown in FIG. 6 may be deformed, as shown in FIG. 8, and may then be detected.

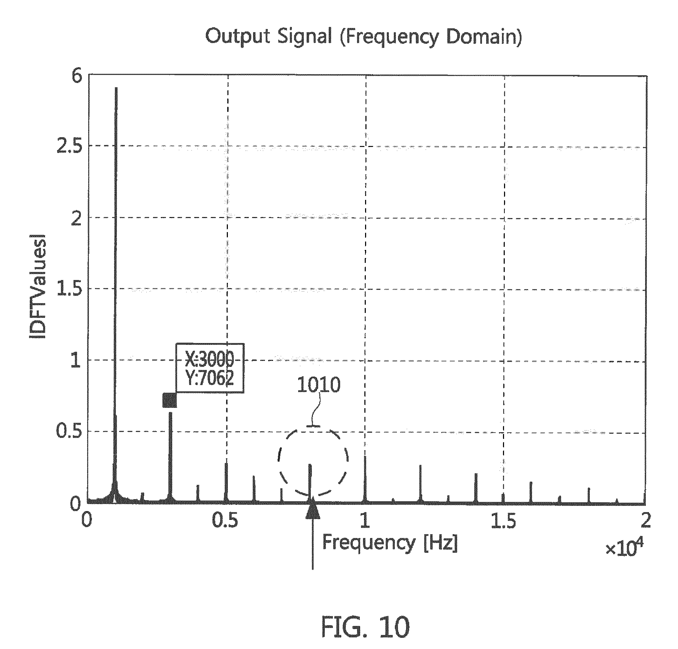

In this case, when the frequency domain of the signal, deformed as shown in FIG. 8, is checked, harmonic peaks 1010 may appear, as shown in FIG. 10.

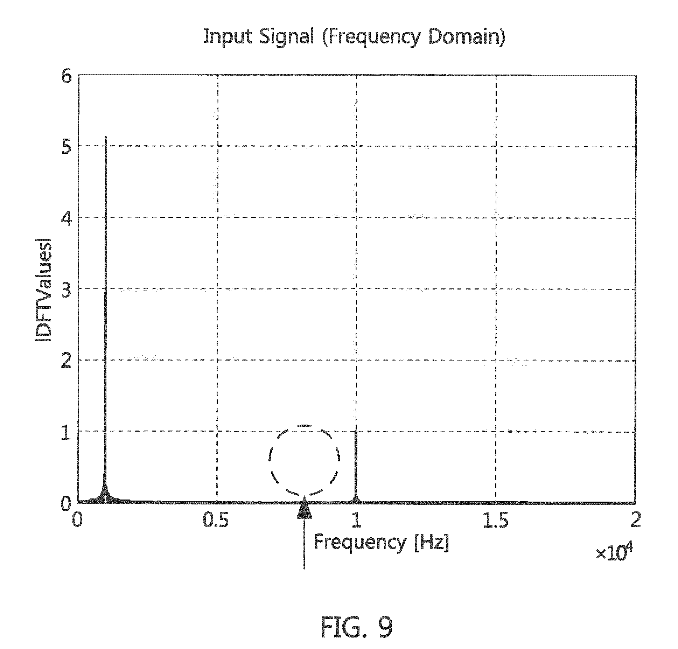

When no nonlinear magnetic particle is present in the sample to be checked, harmonic peaks may not appear, as shown in FIG. 9. At this time, distortion may occur to some degree due to nonlinear elements, such as amplifiers, multipliers, or filters, but these distortion values are known characteristics, and may be removed through a frequency filter.

That is, depending on whether a harmonic peak at a specific frequency has been detected, whether a nonlinear magnetic particle is present in the sample may be determined, and this determination procedure may correspond to the operating principles of the FMMD scheme.

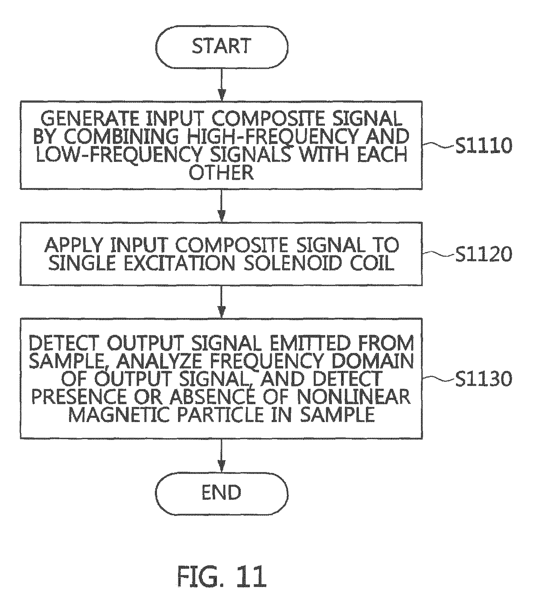

FIG. 11 is an operation flowchart showing a method for detecting a nonlinear magnetic particle based on a single excitation coil according to an embodiment of the present invention.

Referring to FIG. 11, the method for detecting a nonlinear magnetic particle based on a single excitation coil according to the embodiment of the present invention generates an input composite signal by combining a high-frequency sine wave signal and a low-frequency sine wave signal, which are generated based on a fundamental frequency, with each other at step S1110.

Here, the fundamental frequency may correspond to a fundamental waveform that is indicated by a sine function and is used to indicate a spatial frequency or sound.

Here, before the input signal is applied to the excitation solenoid coil, the input composite signal is generated by combining two signals, and thus the procedure for performing calibration using two excitation solenoid coils in the structure of FIG. 1 may be omitted. That is, there is an advantage in that the expense incurred for one excitation solenoid coil and the expense incurred for calibration may be reduced.

In this case, by using a combiner for adding and combining two signals, a high-frequency sine wave signal and a low-frequency sine wave signal may be combined with each other, and then an input composite signal may be generated.

Here, the combiner may be a passive electronic element for adding and combining two signals. That is, by adding and combining two signals, they may be combined with each other without mutually influencing each other.

The combiner may correspond to any one of a Radio Frequency (RF) combiner, corresponding to the characteristics of the input composite signal, a summing amplifier, and electronic equipment enabling an add operation on analog signals.

Here, the RF combiner, which is a kind of passive circuit, may mean a circuit for equally or differentially distributing the power of a specific signal or for combining the power. Here, the RF combiner may add and combine two signals, unlike a mixer for mixing two frequencies with each other and detecting only a frequency signal corresponding to the difference between the two frequencies.

The summing amplifier may be a circuit for performing only an add function, among the functions of an existing operational amplifier for adding or subtracting multiple signals, or differentiating or integrating the signals.

Here, as the combiner of the present invention, which one of the RF combiner and the summing amplifier is to be used may be set by a user or a manager who implements FMMD.

Further, the combiner may be a concept including not only electronic equipment that is conventionally used, such as an RF combiner or a summing amplifier, but also all electronic equipment that may be developed in the future and enables an add operation on analog signals.

Here, the strength of the high-frequency sine wave signal and the strength of the low-frequency sine wave signal are respectively amplified using a first amplifier and a second amplifier, and the amplified sine wave signals are input to the combiner. The strength of the combined signal, output from the combiner, is amplified using a third amplifier, and thus the input composite signal may be generated.

Here, the first amplifier and the second amplifier may adjust and amplify the strengths of the high-frequency sine wave signal and the low-frequency sine wave signal, respectively, depending on the characteristics of the input composite signal. By differently amplifying the strengths of respective signals, the effect of controlling the characteristics of an input signal may be implemented by calibrating the ratio and the geometric positions of two excitation solenoid coils in the conventional technology.

Further, the third amplifier may adjust the strength of the signal in response to loss that occurs in the procedure for combining two signals through the combiner, which is a passive electronic element. That is, the strength of the combined signal output from the combiner may be amplified in response to the loss of strength occurring in the combiner.

Next, the method for detecting a nonlinear magnetic particle based on a single excitation coil according to the embodiment of the present invention applies the input composite signal to the single excitation solenoid coil included in a measurement head, through which the sample required to detect a nonlinear magnetic particle passes, in order to generate a magnetic field in the measurement head at step S 1120.

Here, "nonlinear magnetic particle" may literally mean a material or a particle having a nonlinear magnetic property. That is, the nonlinear magnetic particle may be a material for making a response that is not proportional to the strength or intensity of the magnetic field generated in the single excitation solenoid coil.

Here, the measurement head may be composed of several layers, wherein the single excitation solenoid coil may be located in the outermost layer of the measurement head. Further, the sample may be influenced by the magnetic field generated in the single excitation solenoid coil while passing through the center portion of the measurement head.

Thereafter, the method for detecting a nonlinear magnetic particle based on a single excitation coil according to the embodiment of the present invention detects an output signal, emitted from the sample depending on the magnetic field, using at least one detection solenoid coil included in the measurement head, analyzes the frequency domain of the output signal, and detects whether a nonlinear magnetic particle is present in the sample at step S 1130.

Here, the magnetic field may be the sum of a first magnetic field, generated in response to the high-frequency sine wave signal, and a second magnetic field, generated in response to the low-frequency sine wave signal. That is, in the structure shown in FIG. 1, when two excitation solenoid coils respectively generate magnetic fields and the magnetic fields are added with each other by calibrating the geometric positions of the two coils, the present invention may obtain the same effect that is obtained by applying a signal, in which the two signals are combined with each other, to a single excitation solenoid coil.

Whether a harmonic peak is detected in the frequency domain is checked. When a harmonic peak is detected, it may be determined that a nonlinear magnetic particle is present in the sample.

Such a harmonic peak, which is a frequency peak corresponding to a specific frequency, may be detected when a nonlinear magnetic particle is present in the sample. Here, the characteristics of the corresponding particle may also be grasped based on the harmonic peak detected in the frequency domain.

When a signal in which two frequencies are combined with each other is detected in a deformed shape in the frequency domain, it may be checked whether a harmonic peak is detected. That is, when a nonlinear magnetic particle is present in the sample, the output signal emitted from the sample may be detected in a form that is not proportional to the combined signal of two frequencies.

Therefore, when a deformed signal that is not proportional to the combined signal of two frequencies is detected in this way, it may be inferred that a nonlinear magnetic particle is present in the sample, and the detection of the harmonic peak may be performed.

Here, at least one detection solenoid coil may be located closer to the center of the measurement head than the single excitation solenoid coil in the measurement head. Therefore, the single excitation solenoid coil may be located in the outermost layer of the measurement head, at least one detection solenoid coil may be located further inside than the single excitation solenoid coil, and a space through which the sample passes may be present at the center of the measurement head, which corresponds to an area further inside than the detection solenoid coil.

When FMMD is implemented using the above-described nonlinear magnetic particle detection method, an operation of delicately calibrating the ratio of two excitation solenoid coils, the geometric positions of the coils, etc. may be omitted, thus reducing expenses when products are produced.

Further, when the FMMD for measuring the nonlinearity of a nonlinear material located in the magnetic field applied to the single excitation solenoid coil is implemented, the accuracy of detection may be improved.

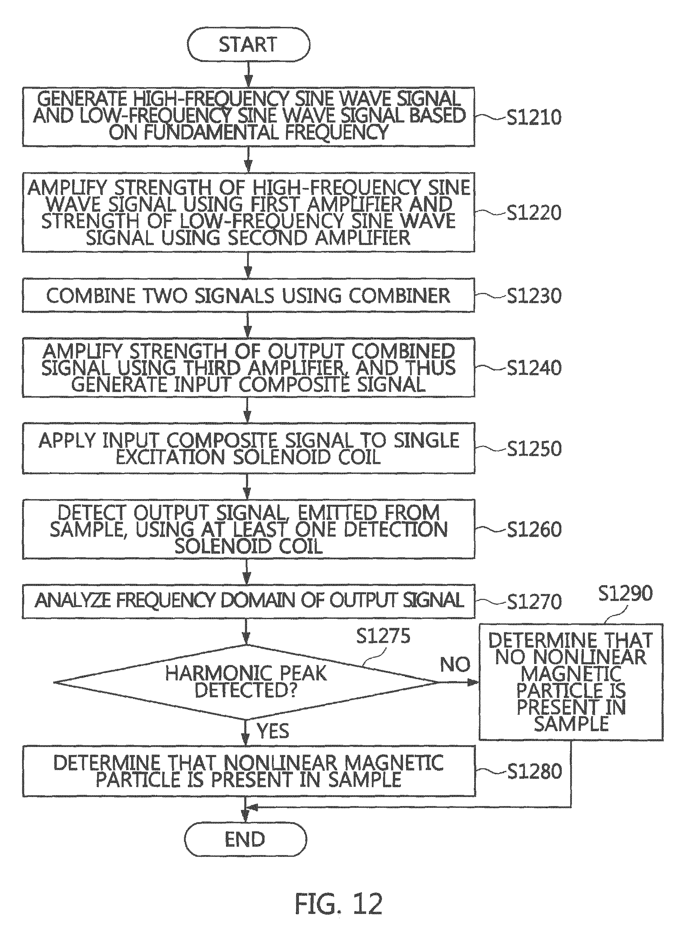

FIG. 12 is an operation flowchart showing in detail a method for detecting a nonlinear magnetic particle based on a single excitation coil according to an embodiment of the present invention.

Referring to FIG. 12, the method for detecting a nonlinear magnetic particle based on a single excitation coil according to the embodiment of the present invention generates a high-frequency sine wave signal and a low-frequency sine wave signal using a fundamental frequency at step S1210.

Thereafter, the strength of the high-frequency sine wave signal is amplified using a first amplifier and the strength of the low-frequency sine wave signal is amplified using a second amplifier at step S1220.

Here, the first amplifier and the second amplifier may amplify the strengths of the respective signals to corresponding levels depending on the characteristics of an input composite signal.

Thereafter, the two signals, the strengths of which have been amplified, are added and combined using a combiner at step S1230.

In this case, the combiner may be any one of devices corresponding to the characteristics of the input composite signal, wherein the devices may include an RF combiner, a summing amplifier, and electronic equipment enabling an add operation on analog signals.

Further, the combiner may be a passive electronic element. Therefore, in the procedure for combining two signals with each other, loss may occur.

Thereafter, the strength of a combined signal output from the combiner is amplified using a third amplifier, and thus an input composite signal is generated at step S1240.

That is, as compensation for the loss occurring in the procedure for combining two signals with each other in the combiner, the strength of the combined signal may be amplified.

Thereafter, the input composite signal is applied to the single excitation solenoid coil at S1250.

Here, the single excitation solenoid coil may generate a magnetic field corresponding to two frequencies.

Thereafter, an output signal emitted from the sample depending on the magnetic field is detected using at least one detection solenoid coil at step S1260.

Next, the frequency domain of the output signal is analyzed at step S1270.

Thereafter, it is determined whether a harmonic peak is detected in the frequency domain at step S1275.

If, as a result of the determination at step S1275, a harmonic peak is detected in the frequency domain, it is determined that a nonlinear magnetic particle is present in the sample at step S1280.

In contrast, if, as a result of the determination at step S1275, a harmonic peak is not detected in the frequency domain, it is determined that no nonlinear magnetic particle is present in the sample at step S1290.

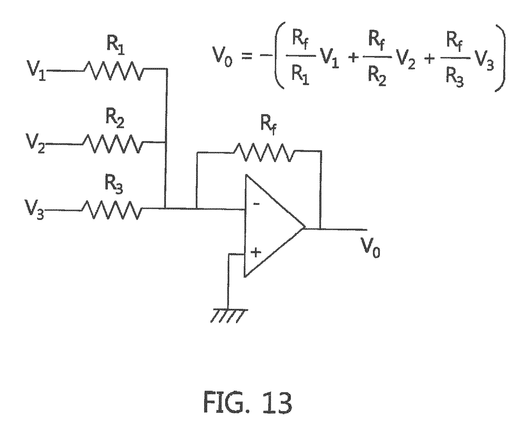

FIG. 13 is a circuit diagram showing an example of a summing amplifier according to an embodiment of the present invention.

Referring to FIG. 13, the summing amplifier of the present invention may correspond to a circuit producing sum of weights for each input.

As described in FIG. 13, the summing amplifier may produce a result by summing each voltage value multiplied by each resistance R_{f} and each ratio of the input resistance Rᵢ. The parity may be inverted in this case.

For example, in FIG. 13, if V₁ corresponds to a high-frequency sine wave signal sin(2*pi*f₁*t) generated by a high-frequency generation module, V₂ corresponds to a low-frequency sine wave sin(2*pi*f₂*t) generated by a low-frequency generation module and V₃ corresponds to 1 which is a DC offset signal, the final output value V₀ corresponds to -{(sin(2*pi*f₁*t)*(R_{f}/R₁)) + (sin(2*pi*f₂*t)*(R_{f}/R₂)) + (1*(R_{f}/R₃))}.

In accordance with the present invention, it is possible to reduce expenses required for the production of products by omitting an operation of delicately calibrating the ratio, geometric positions, or the like of two excitation solenoid coils when FMMD is implemented.

Further, the present invention may improve the accuracy of detection when FMMD for measuring the nonlinearity of a nonlinear material located in a magnetic field applied to a single excitation solenoid coil is implemented.

As described above, in the apparatus and method for detecting a nonlinear magnetic particle based on a single excitation coil according to the present invention, the configurations and schemes in the above-described embodiments are not limitedly applied, and some or all of the above embodiments can be selectively combined and configured so that various modifications are possible.

## Claims

1. An apparatus for detecting a nonlinear magnetic particle based on a single excitation coil, comprising:
a signal generation unit for generating an input composite signal by combining a high-frequency sine wave signal and a low-frequency sine wave signal, generated based on a fundamental frequency, with each other;
a signal application unit for applying the input composite signal to a single excitation solenoid coil included in a measurement head, through which a sample passes, to generate a magnetic field in the measurement head so as to detect a nonlinear magnetic particle; and
a detection unit for detecting an output signal emitted from the sample depending on the magnetic field by using at least one detection solenoid coil included in the measurement head, for analyzing a frequency domain of the output signal, and for detecting whether the nonlinear magnetic particle is present in the sample.

2. The apparatus of claim 1, wherein the magnetic field is a sum of a first magnetic field, generated in response to the high-frequency sine wave signal, and a second magnetic field, generated in response to the low-frequency sine wave signal.

3. The apparatus according to any one of the preceding claims, wherein the detection unit checks whether a harmonic peak is detected in the frequency domain, and determines that the nonlinear magnetic particle is present in the sample if the harmonic peak is detected.

4. The apparatus of claim 3, wherein the detection unit is configured to, when a signal in which two frequencies are combined with each other is detected in a deformed shape in the frequency domain, check whether the harmonic peak is detected.

5. The apparatus according to any one of the preceding claims, wherein the signal generation unit generates the input composite signal by combining the high-frequency sine wave signal with the low-frequency sine wave signal using a combiner that adds and combines two signals.

6. The apparatus of claim 5, wherein the signal generation unit generates the input composite signal in such a way as to amplify a strength of the high-frequency sine wave signal and a strength of the low-frequency sine wave signal using a first amplifier and a second amplifier, respectively, to input the amplified sine wave signals to the combiner, and to amplify a strength of a combined signal output from the combiner using a third amplifier.

7. The apparatus according to claim 5 or 6, wherein the combiner corresponds to any one of a Radio Frequency (RF) combiner, a summing amplifier, and electronic equipment, which correspond to characteristics of the input composite signal, wherein the electronic equipment enables an add operation on analog signals.

8. The apparatus according to any one of the preceding claims, wherein the at least one detection solenoid coil is located in the measurement head at a position closer to a center of the measurement head than the single excitation solenoid coil.

9. A method for detecting a nonlinear magnetic particle based on a single excitation coil, comprising:
generating an input composite signal by combining a high-frequency sine wave signal and a low-frequency sine wave signal, generated based on a fundamental frequency, with each other;
applying the input composite signal to a single excitation solenoid coil included in a measurement head, through which a sample required to detect a nonlinear magnetic particle passes, so as to generate a magnetic field in the measurement head; and
detecting an output signal emitted from the sample depending on the magnetic field by using at least one detection solenoid coil included in the measurement head, analyzing a frequency domain of the output signal, and detecting whether the nonlinear magnetic particle is present in the sample.

10. The method of claim 9, wherein the magnetic field is a sum of a first magnetic field, generated in response to the high-frequency sine wave signal, and a second magnetic field, generated in response to the low-frequency sine wave signal.

11. The method of claim 9 or 10, wherein detecting the output signal comprises:
checking whether a harmonic peak is detected in the frequency domain; and
determining that the nonlinear magnetic particle is present in the sample if the hannonic peak is detected.

12. The method of claim 11, wherein checking whether a harmonic peak is detected is configured to, when a signal in which two frequencies are combined with each other is detected in a deformed shape in the frequency domain, check whether the harmonic peak is detected.

13. The method according to one of claims 9 to 12, wherein generating the input composite signal is configured to generate the input composite signal by combining the high-frequency sine wave signal with the low-frequency sine wave signal using a combiner that adds and combines two signals.

14. The method of claim 13, wherein generating the input composite signal comprises:
amplifying a strength of the high-frequency sine wave signal and a strength of the low-frequency sine wave signal using a first amplifier and a second amplifier, respectively, and inputting the amplified sine wave signals to the combiner; and
amplifying a strength of a combined signal output from the combiner using a third amplifier, thus generating the input composite signal.

15. The method of claim 13 or 14, wherein the combiner corresponds to any one of a Radio Frequency (RF) combiner, a summing amplifier, and electronic equipment, which correspond to characteristics of the input composite signal, wherein the electronic equipment enables an add operation on analog signals.
